# FASCICULE DE BREVET EUROPEEN

(11) **EP 3 241 587 B1**
(45) Date de publication et mention de la délivrance du brevet: **06.03.2019**
(21) Numéro de dépôt: 17169087.8
(22) Date de dépôt: 02.05.2017
(51) Int. Cl.: A23L 33/00, A23L 33/10, A23L 33/125, A23G 3/34, A23G 3/36, A23G 3/52

(54) **COMPOSITION POUR COMPLEMENT ALIMENTAIRE**
ZUSAMMENSETZUNG FÜR NAHRUNGSERGÄNZUNGSMITTEL
COMPOSITION FOR FOOD SUPPLEMENT

(30) Priorité: 04.05.2016 FR 1654087
(43) Date de publication de la demande: 08.11.2017
(73) Titulaire: TAKABIO, 49070 Beaucouze (FR)
(72) Inventeur: FAVREAU, Charlotte, Joséphine, Camille, 49070 Beaucouzé (FR)
(74) Mandataire: Cabinet Netter

(56) Documents cités:
- WO-A2-2010/057188
- CN-A- 101 181 067
- US-A- 6 057 139
- US-B1- 6 365 208
- DATABASE GNPD [Online] MINTEL; août 2003 (2003-08), Anonymous: "F&J Capsule gummy", XP002763591, Database accession no. 221098
- DATABASE GNPD [Online] MINTEL; juin 2014 (2014-06), Anonymous: "Extra strenght chewable tablets for lactose intolerance", XP002763592, Database accession no. 2513649
- DATABASE GNPD [Online] MINTEL; décembre 2015 (2015-12), Anonymous: "Lactase capsules", XP002763593, Database accession no. 3628135
- DATABASE GNPD [Online] MINTEL; mars 2011 (2011-03), Anonymous: "Lactase supplement capsules", XP002763594, Database accession no. 1509610
- DATABASE GNPD [Online] MINTEL; juillet 2013 (2013-07), Anonymous: "Lactase drink", XP002763595, Database accession no. 2092513

## Description

La présente invention se rapporte à une composition pour complément alimentaire sous forme de gomme fondante comprenant une lactase fongique, telle que définie dans les revendications. L'invention se rapporte également à un procédé de préparation dudit complément alimentaire.

Aujourd'hui, il existe un grand nombre de produits issus du lait dans le commerce. Il s'agit de produits dits laitiers. Les produits laitiers sont une source alimentaire très riche en énergie. Certains produits laitiers sont aussi riches en calcium et permettent un bon entretien de la structure osseuse du corps humain. Certaines enzymes du corps humain sont nécessaires à la bonne digestion des produits laitiers. Il s'agit particulièrement de l'enzyme lactase. En effet, la lactase permet d'hydrolyser le lactose qui est un sucre contenu dans les produits laitiers. Le sucre lactose se retrouve également dans de nombreux autres produits de l'industrie agroalimentaire, y compris les biscuits, les viennoiseries, la charcuterie, les soupes, les sauces, ainsi que les condiments et plats cuisinés. Par ailleurs, le lactose est parfois utilisé en tant qu'ingrédient de médicament. Pour la digestion de tous ces produits, le corps humain a donc besoin de lactase.

La lactase permet de dégrader par hydrolyse le lactose (disaccharide) en du galactose (monosaccharide) et du glucose (monosaccharide).

La lactase fait partie de la famille des β-galactosidases.

Certains humains sont déficients en lactase. D'une manière générale, il s'agit du fait que le corps des personnes concernées ne produit pas assez de lactase. Il s'ensuit une incapacité (totale ou partielle) du corps à hydrolyser le lactose en galactose et glucose. La déficience entraîne des pathologies douloureuses lors de la digestion de produits laitiers. Il s'agit notamment de douleurs du tractus gastro-intestinal (nausées, crampes, diarrhées, etc.), après l'ingestion d'un produit comestible contenant du lactose. Les personnes concernées sont alors qualifiées d'intolérantes au lactose. D'une manière générale, on parle d'une « intolérance au lactose » lorsqu'il existe une quelconque difficulté du métabolisme à digérer le lactose présent dans des produits alimentaires.

L'intolérance au lactose affecte un pourcentage significatif de la population humaine. Dans certains cas, l'intolérance au lactose peut résulter en une perte de poids qui peut entraîner des états graves.

Les personnes affectées d'intolérance au lactose tentent généralement de pallier les symptômes douloureux de plusieurs manières. Une manière consiste simplement à éviter les produits laitiers et autres produits contenant du lactose. Mais cela prive la personne de produits riches en énergie et en calcium, ainsi que d'aliments usuels dans la vie quotidienne.

Une autre manière est une consommation de produits laitiers dépourvus de lactose. De tels produits sont aujourd'hui disponibles dans le commerce, mais restent chers en raison du traitement en amont de ces aliments pour éliminer/hydrolyser le lactose initialement présent dans ces produits.

Le document WO 2008/030828 divulgue un produit alimentaire traité avec une enzyme de dégradation de lactose.

Une autre manière encore consiste à ingérer un complément alimentaire par voie orale comprenant une dose efficace de lactase. Ces compléments se présentent classiquement sous forme de gélules, de comprimés ou de poudre. La lactase provient généralement d'une source fongique ou de levures. Toutefois, cette manière reste relativement contraignante, et ce notamment en raison de l'aspect et de la galénique des produits existants (comprimés, gélules, poudres). Par ailleurs, le complément alimentaire doit généralement être absorbé en même temps que la consommation du produit laitier. Ceci peut altérer le goût et/ou l'aspect des produits.

Le document EP 2 120 892 divulgue une composition de particules comprenant de la lactase.

Il existe également des compléments alimentaires à base de lactase provenant de bactéries. Une méthode consiste à ingérer des bactéries ayant une membrane cellulaire poreuse. Les bactéries métabolisent et dégradent alors le lactose au sein des cellules bactériennes. L'ingestion des bactéries doit généralement se faire en même temps que la consommation du produit laitier, ce qui peut altérer le goût et/ou l'aspect des produits. De plus, l'isolation des lactases issues de bactéries et les méthodes biotechnologiques pour générer des bactéries à membrane poreuse sont coûteuses.

La lactase est une enzyme instable à température ambiante (environ 25°C). En effet, la lactase présente dans les compléments alimentaires perd son activité, si bien qu'après un certain temps le complément n'est plus efficace contre l'intolérance au lactose. Ainsi, les produits de l'état de la technique présentent une durée d'utilisation assez brève. Afin de viser une date limite d'utilisation optimale (DLUO) la plus longue, un surdosage d'enzyme est généralement effectué dans les compléments alimentaires à lactase. Ceci génère des problèmes de production industrielle (notamment des surcoûts en raison du surdosage) et de commercialisation.

Le document DATABASE GNPD [Online] MINTEL ; juin 2014 (2014-06), Anonymous : « Extra strengh chewable tablets for lactose intolerance »*, Database accession no.* 2513649 divulgue complément alimentaire de lactase sous forme de comprimé mâchable.

Le document DATABASE GNPD [Online] MINTEL; août 2003 (2003-08), Anonymous : « F&J Capsule gummy »*, Database accession no. 221098* divulgue un complément alimentaire gommeux de vitamine C.

La présente invention vient améliorer la situation.

À cet effet, l'invention vient introduire une composition de complément alimentaire conforme aux revendications ci-jointes. La composition comprend en poids, de 3 % à 8 % d'eau ; de 14 % à 20 % de sirop de glucose ; de 60 % à 80 % de sucre ; de 1 % à 3 % de gélatine ; de 0,1 % à 2 % d'une ou plusieurs substances arômatisantes ; et de 2 % à 6 % de lactase fongique issue de *Aspergillus oryzae* présentant une activité enzymatique de 100000 unités par gramme d'enzyme. La composition de l'invention présente une stabilité enzymatique accrue. De plus, la composition est adaptée pour une ingestion régulière pour palier les effets néfastes d'une intolérance au lactose.

Selon l'invention, le sucre comprend en poids 0,15 % en sucre glace et le reste en sucre cristallisé. Le sucre glace permet une bonne initialisation de la cristallisation lors du procédé d'obtention de la composition de l'invention.

La composition de l'invention peut en outre comprendre de 0,1 % à 2 % de colorant. Cela permet de donner une couleur au produit final et de le rendre plus attractif à la consommation.

Selon l'invention, le complément alimentaire est confectionné sous forme de gomme fondante. La composition de complément alimentaire de l'invention est très stable en termes d'activité enzymatique à température ambiante lorsqu'elle est sous forme de gomme fondante. Plus précisément, l'enzyme est particulièrement stable lorsque le complément alimentaire de l'invention est confectionné sous forme de gomme fondante.

L'invention vise également un procédé de production de complément alimentaire conforme aux revendications ci-jointes. Le procédé comprend les étapes suivantes :
a. Mélanger de l'eau, du sirop de glucose, du sucre et de la gélatine pour obtenir une première formulation ;
b. Ajouter de la lactase fongique à la première formulation de l'étape a. pour obtenir une deuxième formulation, la lactase fongique étant issue de *Aspergillus oryzae* et présentant une activité enzymatique de 100000 unités par gramme d'enzyme ;
c. Réguler en cuve tampon la deuxième formulation obtenue à l'étape b. par ajout de sucre pour obtenir une troisième formulation ;
d. Ajouter une ou plusieurs substances arômatisantes ;
e. Aérer la troisième formulation dans un foisonneur de manière à former une gomme fondante ;
f. Sécher ladite gomme fondante par évaporation d'une partie de l'eau ;
dans lequel les quantités en poids de l'eau, du sirop de glucose, du sucre, de la gélatine, de la lactase fongique et de la ou les substances arômatisantes dans les étapes a. à d. sont choisies de manière à obtenir une composition pour complément alimentaire, après le séchage de l'étape f., comportant de 3 % à 8 % d'eau, de 14 % à 20 % de sirop de glucose, de 60 % à 80 % de sucre, de 1 % à 3 % de gélatine, de 0,1 % à 2 % d'une ou plusieurs substances arômatisantes, et de 2 % à 6 % de lactase fongique.

L'étape a. du procédé pour l'obtention de la première formulation peut comprendre les sous-étapes suivantes :
a1. Mélanger une partie de l'eau et une partie du sirop de glucose à une température inférieure ou égale à 80°C ;
a2. Ajouter au mélange de l'étape a1. du sucre et mélanger à une température d'environ 80°C;
a3. Ajouter le restant du sirop de glucose au mélange de l'étape a2. et mélanger à une température comprise entre 80°C et 100°C ;
a4. Mélanger la gélatine avec le restant de l'eau à une température d'environ 95°C ;
a5. Ajouter le mélange obtenu à l'étape a4. au mélange obtenu à l'étape a3. à une température d'environ 90°C.

Ceci permet d'avoir de bonnes conditions d'homogénéité du produit final.

L'ajout de la lactase fongique à l'étape b. afin d'obtenir la deuxième formulation est réalisé de préférence à une température d'environ 45°C ou inférieure à 45°C. Ceci permet de garder l'intégrité et l'activité de l'enzyme. En effet, l'enzyme de l'invention peut être instable au-delà de 45°C.

La régulation à l'étape c. comprend de préférence l'ajout de sucre glace. Comme mentionné plus haut, cela permet une bonne initialisation de la cristallisation du produit final.

Selon un mode de réalisation qui vise la production d'un produit final avec différents goûts, l'étape d. d'aromatisation peut être réalisée après l'étape e. d'aération. Dans un autre mode de réalisation qui vise un produit final avec un seul goût, l'étape d. d'aromatisation peut être réalisée avant l'étape e. d'aération, à savoir sensiblement à l'étape b. d'ajout de lactase. Dans ce mode de réalisation, l'étape b. comprend l'étape d. d'aromatisation. La substance arômatisante est donc ajoutée en parallèle de la lactase à la première formulation de l'étape a. ; lors de l'étape de régulation c., la deuxième formulation comprend donc déjà l'arôme.

L'étape f. de séchage peut comprendre en outre une étape f1. qui consiste à confectionner la troisième formulation par une ou plusieurs opérations choisies parmi le moulage/démoulage dans des moules d'amidon, le coulage, le séchage et le dépoudrage. Ceci permet de donner l'aspect et l'apparence souhaités au complément alimentaire de l'invention.

En outre, le procédé de l'invention peut comprendre une étape de stockage qui consiste à stocker la troisième formulation. Le stockage permet notamment d'organiser la distribution commerciale adéquate. Le stockage est possible en raison de la stabilité enzymatique accrue de la composition de l'invention.

D'autres avantages et caractéristiques de l'invention apparaîtront à la lecture de la description détaillée ci-après et sur les dessins annexés, sur lesquels :
- La figure 1 représente un organigramme d'un procédé de fabrication de la composition selon l'invention ;
- La figure 2 représente l'effet du pH par rapport à l'activité enzymatique d'une lactase mise en oeuvre dans l'invention ;
- La figure 3 représente l'effet du pH par rapport à la stabilité d'une lactase mise en oeuvre dans l'invention ;
- La figure 4 représente l'effet de la température par rapport à l'activité enzymatique d'une lactase mise en oeuvre dans l'invention ;
- La figure 5 représente l'effet de la température par rapport à la stabilité d'une lactase mise en oeuvre dans l'invention ; et
- La figure 6 montre une photo d'une vue de dessus et une photo d'une vue de dessous d'un complément alimentaire selon l'invention.

Les dessins et la description ci-après contiennent, pour l'essentiel, des éléments de caractère certain. Ils font partie intégrante de la description, et pourront donc non seulement servir à mieux faire comprendre la présente invention, mais aussi contribuer à sa définition, le cas échéant.

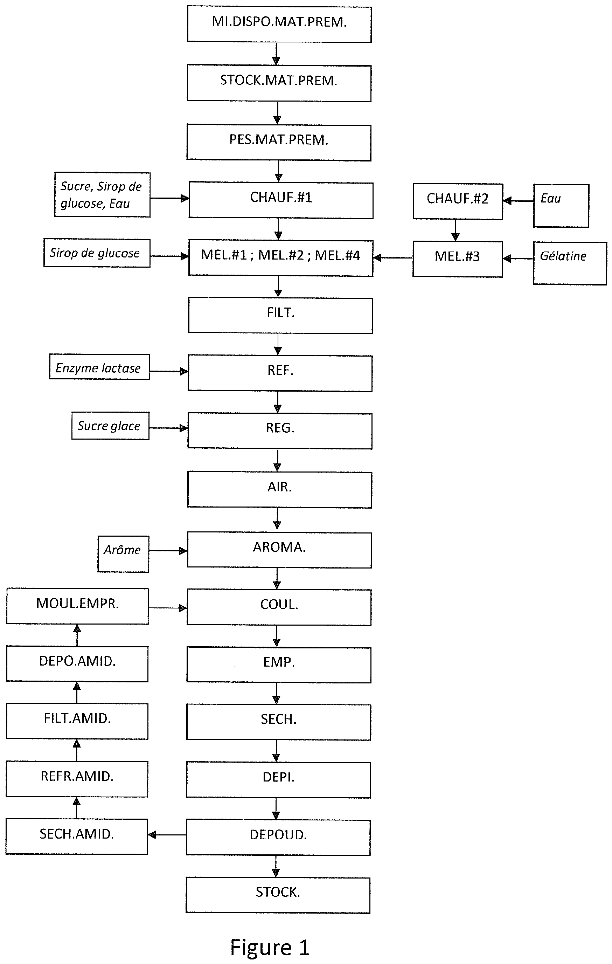

La figure 1 montre un organigramme du procédé de fabrication d'une composition pour complément alimentaire selon l'invention.

Une première opération MI.DISPO.MAT.PREM. de mise à disposition de matières premières permet de rassembler les ingrédients nécessaires pour la préparation de la composition selon l'invention. Il s'agit des ingrédients constitutifs du complément alimentaire final.

Ainsi, l'opération MI.DISPO.MAT.PREM comprend la mise à disposition d'eau, de sirop de glucose, de sucre, de gélatine, d'une ou plusieurs substances arômatisantes et de lactase fongique issue de *Aspergillus oryzae.* La lactase fongique selon l'invention présente une activité enzymatique d'environ 100000 unités par gramme d'enzyme.

L'opération MI.DISPO.MAT.PREM. peut comprendre le contrôle qualitatif des ingrédients de la composition pour complément alimentaire. En ce sens, il s'agit notamment de contrôler la pureté et la qualité de chaque ingrédient, et tout particulièrement de tester l'activité enzymatique souhaitée de la lactase fongique fixée de préférence à 100000 unités par gramme d'enzyme.

La première opération est suivie d'une opération STOCK.MAT.PREM. de stockage des matières premières. L'opération STOCK.MAT.PREM. comprend le stockage de chaque ingrédient dans des conditions adéquates. La lactase fongique est avantageusement stockée à environ 4°C afin de ne pas perdre d'activité enzymatique.

L'opération STOCK.MAT.PREM. de stockage des matières premières est généralement suivie d'une opération PES.MAT.PREM. de pesée desdits ingrédients (matières premières).

L'opération PES.MAT.PREM. permet notamment de choisir la bonne quantité de chaque ingrédient de manière à obtenir les pourcentages en poids souhaités de la composition pour complément alimentaire.

Selon l'invention, les quantités en poids de l'eau, du sirop de glucose, du sucre, de la gélatine, de la lactase fongique et de la ou des substance(s) arômatisante(s) sont choisies de manière à obtenir une composition dans des proportions précises. Pour cela, il faut considérer que le procédé de préparation de l'invention comprend une étape dans laquelle une partie de l'eau employée est évaporée. Ainsi, les quantités des ingrédients employés dans le procédé de l'invention sont choisies de sorte que le produit final est une composition comportant de 3 % à 8 % d'eau, de 14 % à 20 % de sirop de glucose, de 60 % à 80 % de sucre, de 1 % à 3 % de gélatine, de 0,1 % à 2 % d'une ou plusieurs substance(s) arômatisante(s), et de 2 % à 6 % de lactase fongique.

Dans un mode de réalisation, la composition de l'invention est constituée de 3 % à 8 % d'eau, de 14 % à 20 % de sirop de glucose, de 60 % à 80 % de sucre, de 1 % à 3 % de gélatine, de 0,1 % à 2 % d'une ou plusieurs substance(s) arômatisante(s), et de 2 % à 6 % de lactase fongique.

Dans un autre mode de réalisation, la composition de l'invention comporte 3,5 % à 8 % d'eau, de 15,3 % à 20 % de sirop de glucose, de 69 % à 75 % de sucre, de 1,5 % à 1,7 % de gélatine, de 0,1 % à 0,45 % d'une ou plusieurs substance(s) arômatisante(s), et de 2,25 % à 6 % de lactase fongique. Dans encore un autre mode de réalisation, la composition de l'invention est constituée de 3,5 % à 8 % d'eau, de 15,3 % à 20 % de sirop de glucose, de 69 % à 75 % de sucre, de 1,5 % à 1,7 % de gélatine, de 0,1 % à 0,45 % d'une ou plusieurs substance(s) arômatisante(s), et de 2,25 % à 6 % de lactase fongique.

L'opération PES.MAT.PREM. est suivie d'une première opération de chauffage CHAUF.#1 dans laquelle une partie de l'eau et une partie du sirop de glucose sont chauffées à une température sensiblement inférieure ou égale à 80°C. Cette opération CHAUF.#1 est suivie d'une opération MEL.#1 dans laquelle la partie de l'eau et la partie de sirop de glucose sont mélangées à du sucre à une température sensiblement égale à 80°C. Les opérations CHAUF.#1 et MEL.#1 permettent notamment de contrôler l'homogénéité du mélange, ainsi que la consistance du mélange. L'opération MEL.#1 est suivie d'une deuxième opération de mélange MEL.#2 dans laquelle le restant de sirop de glucose est ajouté. L'opération de mélange MEL.#2 est réalisée à une température comprise entre 80°C et 100°C. Parallèlement aux opérations CHAUF.#1, MEL.#1 et MEL.#2, une opération CHAUF.#2 porte une partie de l'eau à une température sensiblement égale à 95°C. L'opération CHAUF.#2 est suivie d'une opération MEL.#3 dans laquelle la gélatine et l'eau préchauffée sont mélangées. Une opération subséquente de mélange MEL.#4 comprend la combinaison du mélange gélatine/eau et du mélange réalisé par la succession des opérations CHAUF.#1, MEL.#1 et MEL.#2. L'opération MEL.#4 est réalisée à une température sensiblement égale à 90°C.

Optionnellement, les opérations précédentes sont suivies d'une opération FILT. de filtrage.

L'opération FILT. de filtrage peut servir de premier point de contrôle [premier point de contrôle critique : CCP 1; en anglais : *Critical Control Point*]. L'opération de filtrage permet notamment d'éliminer tout corps étranger du mélange et/ou d'obtenir un mélange homogène.

L'opération REF. de refroidissement suivante consiste à laisser refroidir (activement ou passivement) le mélange et d'ajouter la lactase fongique aux ingrédients déjà mélangés. Cette opération REF. aboutit à une température sensiblement égale à 45°C. Cette température permet d'une part le bon mélange à chaud (notamment pour une bonne homogénéité du mélange), et d'autre part de ne pas dénaturer la lactase qui est une enzyme thermosensible. L'opération REF. de refroidissement est réalisée dans une cuve de refroidissement.

L'opération REF. est suivie d'une opération REG. de régulation. L'opération REG. comprend l'ajout de sucre sous forme de sucre glace dans le mélange réalisé. Cette opération REG. est réalisée dans une cuve tampon. L'ajout de sucre glace permet d'initialiser la cristallisation de la composition de l'invention. L'ajout de sucre glace à une température de 45°C permet de garder les fins cristaux du sucre glace intacts (les cristaux ne fondent pas à cette température). On évite ainsi des agglomérats de sucre glace et ainsi un produit final hétérogène.

L'opération REG. est suivie d'une opération AIR. d'aération. Cette opération AIR. comprend une aération du mélange dans un foisonneur. Un foisonneur est un appareil qui permet de foisonner le mélange, c'est-à-dire notamment d'alléger et d'augmenter le volume du mélange. Le foisonneur mis en oeuvre dans l'opération AIR. d'aération peut notamment être le modèle B25 de MONDOMIX (marque déposée).

Le fait de foisonner le mélange dans une situation d'aération permet d'obtenir une pâte précurseur de la composition pour complément alimentaire de l'invention. Cette pâte précurseur permet par la suite d'obtenir un produit final en gomme fondante. La gomme fondante présente un aspect meringué. Il peut être fait référence à la gomme fondante sous le nom de « petit Jésus en sucre » ou de « mousse sucrerie » (en anglais : *candy foam*).

La Demanderesse a découvert, non sans surprise, que la composition de l'invention présente une stabilité de l'activité enzymatique accrue par rapport aux produits de l'état de la technique. En effet, la composition sous forme de gomme fondante présente une meilleure stabilité dans le temps par rapport à des formes de types gélule ou comprimé, par exemple.

Il n'est, à ce jour, pas établi avec certitude à quoi est dû cet effet surprenant de stabilité accrue. La Demanderesse formule toutefois deux hypothèses. Chaque hypothèse est basée sur le fait que l'eau (ou l'humidité) déstabilise généralement les enzymes, cf. Effects of water on enzyme performance with an emphasis on the reaction in supercritical fluids, Rezaei K., et al., Crit. Rev. Biotechnol., 2007.

La première hypothèse vise la gélatine et le saccharose présent dans la composition de l'invention. En effet, la gélatine et le saccharose sont des agents dépresseurs de l'activité de l'eau. L'activité de l'eau représente la pression de vapeur d'eau *p* d'un produit humide divisée par la pression de vapeur saturante *p₀* à température identique. Ainsi, la gélatine et le saccharose ont tendance à se lier à l'eau. Cela pourrait faire écran entre l'eau et l'enzyme (réduire la quantité d'eau disponible vis-à-vis de l'enzyme). Cette limitation en eau pourrait être à l'origine d'une activité enzymatique plus stable dans le temps.

La deuxième hypothèse vise l'activité secondaire de certaines enzymes. En effet, certaines enzymes (telles que quelques certaines lactases) possèdent non seulement une activité principale de dégradation du lactose, mais également une faible activité secondaire d'invertase. L'invertase est une réaction de dégradation du saccharose en glucose et fructose. Cette réaction consomme de l'eau. Ainsi, la quantité d'eau est diminuée et il s'ensuit une stabilité accrue de l'enzyme.

Néanmoins, aucune desdites première et deuxième hypothèses n'a pu être vérifiée à ce jour.

De plus, le sucre présent dans la composition de l'invention peut contribuer à la stabilisation de la lactase.

En tout état de cause, les tests de stabilité enzymatique ont montré que le confectionnement sous forme de gomme fondante de la composition de l'invention permet de garder une activité enzymatique appropriée dans le temps. En ce sens, l'activité enzymatique de la composition pour complément alimentaire sous forme de gomme fondante est supérieure dans le temps par rapport aux confectionnements connus, et ce dans des conditions de conservation identiques.

L'opération AIR. d'aération est suivie d'une opération AROMA. qui consiste ici à aromatiser avec plusieurs substances arômatisantes la pâte obtenue dans l'opération AIR. d'aération.

Optionnellement, une opération de coloration peut avoir lieu avant ou après l'opération AIR. d'aération. Dans l'opération de coloration, un ou plusieurs colorants sont ajoutés au mélange afin d'obtenir une ou plusieurs couleurs du produit final.

La pâte précurseur obtenue à l'opération AIR. d'aération est ensuite séchée lors d'une opération SECH. de séchage. L'opération SECH. de séchage permet d'évaporer une partie de l'eau. Dans le mode de réalisation décrit ici, cette opération SECH. de séchage est accompagnée d'un confectionnement. Ainsi, l'opération SECH. est précédée par une opération COUL. de coulage. Cette opération COUL. de coulage consiste à couler la pâte précurseur dans les moules d'amidon à l'aide de trémies. L'opération COUL. de coulage est réalisée à une température sensiblement égale à 45°C. Cette opération COUL. permet notamment de donner la forme voulue (par exemple la forme de dragée) à la pâte précurseur et ainsi, par la suite, au produit final. Des rangées (ou racks) de moules d'amidon peuvent ensuite être empilées les unes sur les autres dans une opération EMP. d'empilement. L'opération EMP. d'empilement permet d'entreprendre un deuxième point de contrôle qui permet en particulier d'éliminer des corps étrangers (par exemple, des débris de bois issus du transport/empaquetage d'ingrédient, etc.).

Les rangées empilées ou les moules d'amidon sont ensuite soumis à l'opération SECH. de séchage, qui peut notamment consister en la mise des rangées/moules à une température inférieure à 40°C (en général autour de 30°C-35°C) pendant un à six jours. Lors de l'opération SECH. de séchage, une partie de l'eau contenu dans le mélange est évaporée. L'opération SECH. de séchage est importante pour obtenir la composition pondérale souhaitée de l'invention.

L'opération SECH. de séchage est suivie d'une opération DEPI. de dépilement desdits rangées/moules d'amidon. Ce dépilement peut être accompagné d'un troisième point de contrôle qui comprend notamment l'inspection des rangées/moules (en particulier pour l'élimination de tout corps étranger). L'opération DEPI. de dépilement est suivie d'une opération DEPOUD. de dépoudrage. L'opération DEPOUD. de dépoudrage a pour effet de séparer la composition finale de l'amidon issu des moules. En effet, l'opération COUL. comprend le coulage de la gomme fondante dans des moules d'amidon. Lorsque la gomme fondante est démoulée desdits moules d'amidon, une certaine quantité d'amidon reste apposée sur la composition. Il convient de l'éliminer.

L'amidon peut être traité et recyclé pour reformer de nouveaux moules d'amidon. Dans le mode de réalisation décrit en figure 1, ce recyclage consiste en une première opération SECH.AMID. de séchage d'amidon, suivie d'une opération REFR.AMID. de refroidissement de l'amidon. Le séchage de l'amidon est généralement réalisé entre 60°C et 70°C. Le refroidissement de l'amidon est généralement réalisé entre 35°C et 40°C. L'amidon est ensuite filtré dans une opération FILT.AMID. afin de le purifier. L'opération FILT.AMID. peut être accompagnée d'un quatrième point de contrôle [deuxième point de contrôle critique : CCP 2]. L'amidon purifié est ensuite déposé et moulé dans deux opérations successives, respectivement l'opération DEPO.AMID pour déposer l'amidon sur des plateaux et l'opération MOUL.EMPR. de moulage d'empreintes. Le recyclage de l'amidon peut être effectué à plusieurs reprises pour la production de moules d'amidon. Le recyclage de l'amidon permet d'établir une boucle entre l'opération DEPOUD. et l'opération COUL. Ainsi, la totalité de la gomme fondante peut être mise sous une forme voulue, par exemple sous la forme de compléments alimentaires en gomme fondante.

Une opération subséquente STOCK. de stockage consiste en le stockage desdits compléments alimentaires en gomme fondante.

D'autres opérations de conditionnement, de mise en carton et/ou de logistique peuvent bien évidemment être prévues après l'opération STOCK. de stockage.

Il est décrit ici que l'ajout au mélange de la substance arômatisante est réalisé après l'opération REF. de refroidissement, et plus précisément après l'opération AIR. d'aération. Ceci est en effet avantageux lorsqu'une pluralité de goûts (au moyen de différentes substances arômatisantes) est prévue pour le produit final. En variante, lorsqu'un goût unique est prévu pour le produit final, il est possible d'ajouter l'arôme souhaité au même moment que l'ajout de la lactase dans la cuve de refroidissement (donc lors de l'opération REF. de refroidissement). L'opération d'aromatisation est donc réalisée avant le foisonnement.

D'une manière plus générale, le procédé de production de complément alimentaire comprend les étapes suivantes :
a. Mélanger de l'eau, du sirop de glucose, du sucre et de la gélatine pour obtenir une première formulation ;
b. Ajouter de la lactase fongique à la première formulation de l'étape a. pour obtenir une deuxième formulation, la lactase fongique étant issue de *Aspergillus oryzae* et présentant une activité enzymatique de 100000 unités par gramme d'enzyme ; cette étape d'ajout de lactase fongique est de préférence réalisée à une température inférieure à 45°C afin de ne pas dégrader l'enzyme ;
c. Réguler en cuve tampon la deuxième formulation obtenue à l'étape b. par ajout de sucre pour obtenir une troisième formulation ; cette étape comprend de préférence l'ajout de sucre glace pour initialiser la cristallisation du produit final ;
d. Ajouter une ou plusieurs substances arômatisantes ;
e. Aérer la troisième formulation dans un foisonneur de manière à former une gomme fondante ;
f. Sécher ladite gomme fondante par évaporation d'une partie de l'eau ;
dans lequel les quantités en poids de l'eau, du sirop de glucose, du sucre, de la gélatine, de la lactase fongique et de la ou les substances arômatisantes dans les étapes a. à d. sont choisies de manière à obtenir une composition pour complément alimentaire après le séchage de l'étape f. comportant 3 % à 8 % d'eau, 14 % à 20 % de sirop de glucose, 60 % à 80 % de sucre, 1 % à 3 % de gélatine, 0,1 % à 2 % d'une ou plusieurs substances arômatisantes, et 2 % à 6 % de lactase fongique.

Ce procédé permet d'obtenir un complément alimentaire sous forme de gomme fondante comprenant en poids 3 % à 8 % d'eau ; 14 % à 20 % de sirop de glucose ; 60 % à 80 % de sucre ; 1 % à 3 % de gélatine ; 0,1 % à 2 % d'une ou plusieurs substances arômatisantes ; et 2 % à 6 % de lactase fongique issue de *Aspergillus oryzae* présentant une activité enzymatique de 100000 unités par gramme d'enzyme.

Concernant le sucre, le complément alimentaire comprend en poids 0,15 % en sucre glace et le reste en sucre cristallisé. Le sucre glace permet d'initier la cristallisation lors de l'opération REG. de régulation.

De manière générale, l'étape a. pour obtenir la première formulation peut comprendre des sous-étapes. Notamment, l'étape a. peut comprendre les étapes de :
a1. Mélanger une partie de l'eau et une partie du sirop de glucose à une température inférieure ou égale à 80°C ;
a2. Ajouter au mélange de l'étape a1. du sucre et mélanger à une température d'environ 80°C;
a3. Ajouter le restant du sirop de glucose au mélange de l'étape a2. et mélanger à une température comprise entre 80°C et 100°C ;
a4. Mélanger la gélatine avec le restant de l'eau à une température d'environ 95°C ;
a5. Ajouter le mélange obtenu à l'étape a4. au mélange obtenu à l'étape a3. à une température d'environ 90°C.

L'étape d. d'aromatisation peut être réalisée après l'étape e. d'aération. L'étape d. d'aromatisation peut aussi être réalisée avant l'étape e. d'aération, à savoir sensiblement en même temps que l'étape b. d'ajout de lactase.

L'étape f. de séchage peut en outre comprendre une étape f1., qui consiste en le confectionnement de ladite troisième formulation par une ou plusieurs opérations choisies parmi le moulage/démoulage dans des moules en amidon, le coulage, le séchage et le dépoudrage.

Le procédé peut aussi comprendre une étape de stockage de ladite troisième formulation.

### EXEMPLE DE RÉALISATION

Un complément alimentaire a été préparé dans les conditions expérimentales décrites ci-avant (cf. procédé indiquant les séquences de mélange et les températures). Un mélange a été réalisé avec les ingrédients en quantités suivantes (% en poids) :
- Eau : 15,96 % ;
- Sirop de glucose : 15,1 % ;
- Sucre (sucre cristallisé et sucre glace) : 63,0 % ;
- Gélatine : 1,40 % ;
- Arôme : 0,34 % ;
- Lactase fongique : 4,20 %

Le sirop de glucose peut être celui le *Glucor 68-80 HM* de Chamtor du groupe VIVESCIA (marque déposée). Le sucre cristallisé provient de la société BRITISH SUGAR PLC. Et le sucre glace provient de la société ISCAL SUGAR. La gélatine provient de la société ITALGELATINE S.p.A. sous la référence produit 250AH30. La gélatine a une force de 250 bloom.

La lactase fongique mise en oeuvre est issue de *Aspergillus oryzae* et présente une activité enzymatique de 100000 unités par gramme d'enzyme. La lactase peut-être obtenue auprès de la société SHIN NIHON CHEMICAL sous la référence produit SUMIZYME (marque déposée) FUNGAL LACTASE 100000 u/g (ou Sumizyme GLL ou Sumizyme FUNGAL LACTASE-SD).

La lactase est une β-Galactosidase (bêta-galactosidase) dont le numéro EC est : EC 3.2.1.23. Ce numéro EC est défini dans la classification des enzymes établie par l'union internationale de biochimie et de biologie moléculaire (IUBMB : *International Union of Biochemistry and Molecular Biology*).La lactase se présente sous forme de poudre sèche fine de couleur blanchâtre. La lactase est inodore et soluble dans l'eau. Cela permet des prédispositions adéquates pour la préparation du complément alimentaire.

La lactase mise en oeuvre présente une activité β-galactosidase pour convertir le lactose en du galactose et du glucose. Le test d'activité enzymatique résulte en : FCC 100000 u/g (*Food Chemicals Codex*). Ce test est basé sur une hydrolyse pendant 15 minutes d'un substrat o-nitrophényle-β-D-galactopyranoside à une température de 37°C et à un pH de 4,5. Ce test est en conformité avec les dispositions du FCC IV, cf. Fourth Edition of the Food Chemicals Codex, Comitee on Food Chemicals Codex, Food and nutrition board, Institute of Medecine, National Academy of Sciences, 1996.

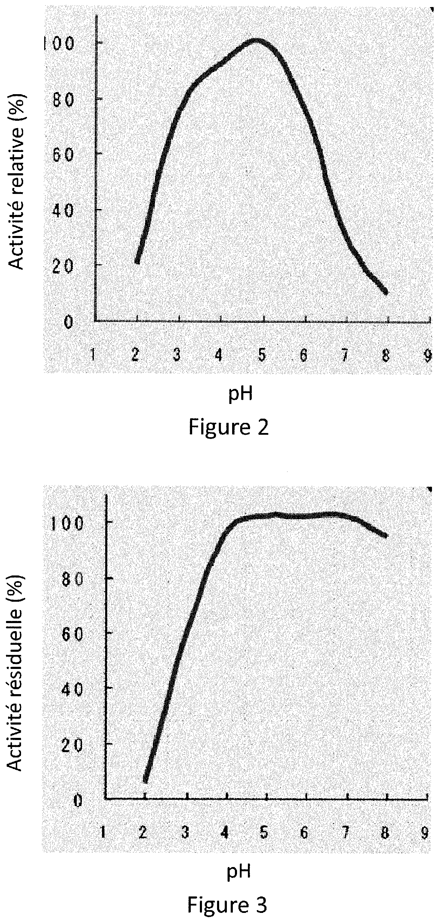

La figure 2 montre l'effet du pH par rapport à l'activité enzymatique de la lactase. La température de réaction est de 37°C. Le temps de réaction est de 10 minutes. Le point optimum du pH se situe à environ pH=4,5.

La figure 3 montre l'effet du pH sur la stabilité de la lactase. La concentration enzymatique est de 1 %. La température de réaction est de 37°C. Le temps d'incubation est de 1 heure. La lactase présente une bonne stabilité pour un pH compris entre pH=4,5 et pH=8.

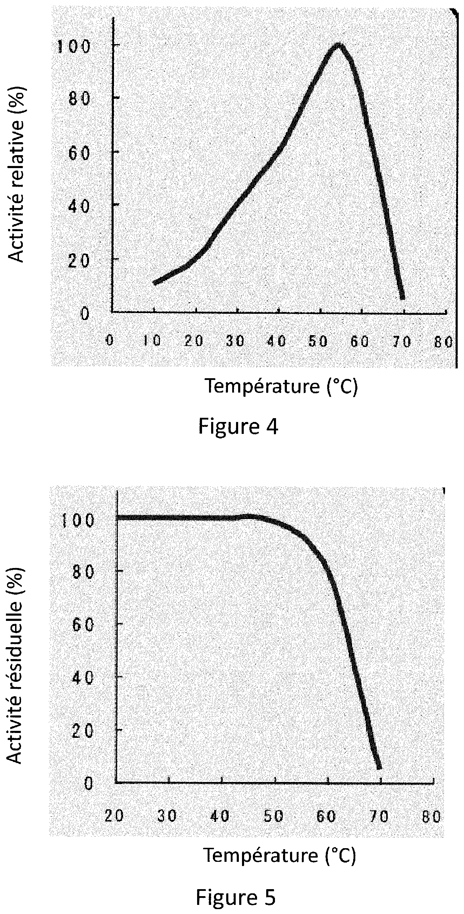

La figure 4 montre l'effet de la température sur l'activité enzymatique de la lactase. Le pH de la réaction est de pH=4,5. Le temps de réaction est de 10 minutes. Le point optimum de température se situe à environ 50°C.

La figure 5 montre l'effet de la température sur la stabilité de la lactase. La concentration enzymatique est de 1 %. Le temps d'incubation est de 30 minutes. La lactase est stable jusqu'à une température d'environ 50°C. L'inactivation de la lactase peut être obtenue en soumettant l'enzyme à une température d'environ 80°C pendant 15 min.

Dans le présent exemple, l'ajout de la lactase fongique est réalisé à une température de 45°C. Ceci permet d'assurer une bonne activité et stabilité de l'enzyme.

L'évaporation de l'eau est réalisée par séchage à une température comprise entre 20 et 40°C pendant une durée de 24h à 6 jours dans des moules réalisés en amidon.

Après l'évaporation d'une partie de l'eau, le mélange présente les quantités suivantes des ingrédients (% en poids):
- Eau : 4,98 % ;
- Sirop de glucose : 17,1 % ;
- Sucre (sucre cristallisé et sucre glace) : 71,2 % ;
- Gélatine : 1,58 % ;
- Arôme : 0,39 % ;
- Lactase fongique : 4,75 %

Les moules d'amidon utilisés lors des opérations de séchage permettent d'obtenir un produit final sous forme de pastilles sensiblement rondes (type dragée). Le diamètre d'une pastille est sensiblement compris entre 15 mm et 25 mm, de préférence environ 20 mm. Le poids d'une pastille est sensiblement compris entre 1,0 g et 1,5 g, de préférence environ 1,3 g.

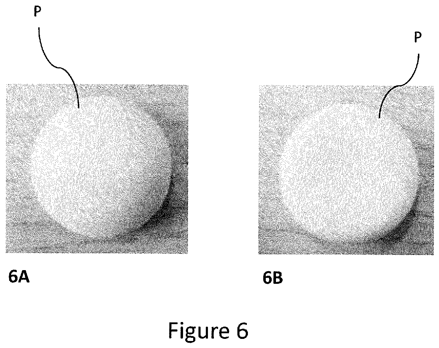

La figure 6 montre une pastille P de gomme fondante du présent exemple de réalisation ayant la composition de l'invention. Le côté gauche 6A de la figure montre une vue de dessus de la pastille P, et le côté droit 6B de la figure une vue de dessous de la pastille P.

Les pastilles à gomme fondante selon l'invention ont été comparées à des produits à lactase sous forme de comprimés et de gélules.

Chaque produit à lactase comparatif comprend la même lactase que la composition de l'invention, à savoir, ici la SUMIZYME FUNGAL LACTASE à 100000 u/g. Les comprimés de lactase ont été réalisés par une technique classique de réalisation de comprimés (mélange des ingrédients nécessaires suivi d'une compression par une comprimeuse). Il en résulte un comprimé de forme oblong (13x8.7mm) d'environ 450 mg. La composition du comprimé est la suivante : maltodextrine ; lactase 100000 FCC/g ; dextrose ; cellulose microcristalline (agent de charge);et stéarate de magnésium (anti-agglomérant).

Les gélules de lactase ont été réalisés par une technique classique de mise en gélule (géluleuse) du mélange d'ingrédients suivant : maltodextrine et lactase 100000 FCC/g. Les gélules utilisées sont des gélules végétales (HPMC) de taille 1, transparentes, avec un poids cible de remplissage de 250mg

Chaque produit a été soumis à un test d'activité enzymatique. Le test tient également compte de mesures d'activité enzymatique effectuées sur un témoin, à savoir la poudre lactase fongique brute (issue de *Aspergillus oryzae* et présentant une activité enzymatique de 100000 unités par gramme d'enzyme). Les produits (y compris le témoin) ont été emballés séparément dans des sacs alimentaires respectifs (non hermétiques), puis stockés en étuve à 25°C / 60% HR (humidité relative). Le test comprend plusieurs mesures d'activité enzymatique, à savoir une mesure respectivement à 2 mois, à 4 mois et à 6 mois. Les mesures d'activité sont réalisés selon la même méthode que précédemment, (méthode SNC). Les résultats figurent dans le tableau 1 ci-dessous :

**Tableau 1: Pourcentage d'activité enzymatique résiduelle par gramme de produit (g) en fonction du temps.**

| **Produit** | **At₀ (%) : Activité Initiale** | **At₁ (%) : Activité après 2 mois** | **At₂ (%) : Activité après 4 mois** | **At₃ (%) : Activité après 6 mois** |
|---|---|---|---|---|
| Gélules à lactase | 100 | 83,6 | 69,4 | 66,4 |
| Comprimés à lactase | 100 | 86,0 | 73,4 | 67,9 |
| Témoin | 100 | 93,7 | 68,0 | 61,6 |
| Pastille (invention) | 100 | 93,5 | 92,0 | 83,9 |

Le test comparatif montre que la composition selon l'invention réalisée sous forme de gomme fondante présente une stabilité bien au-delà des produits comparatifs (gélules et comprimés).

La stabilité de l'activité enzymatique de la lactase contenue dans le produit final présente des avantages. Tout particulièrement, la stabilité dans le temps de la composition de l'invention offre des DLUO tardives des produits finaux, ce qui est très intéressant en termes d'industrialisation et de commercialisation. Grâce à la grande stabilité de la composition de l'invention, une quantité moindre en lactase est nécessaire pour obtenir des DLUO identiques par rapport aux compléments alimentaires de lactase existants. Il n'y a plus besoin de pallier la perte d'activité enzymatique par un surdosage en lactase.

L'exemple de réalisation a été confectionné sous forme de pastilles. D'autres réalisations sont envisageables. Notamment, il existe une grande variabilité de formes du produit final. Ainsi, le complément alimentaire en gomme fondante peut se présenter, par exemple, sous forme de perles ou toute autre forme attractive à la consommation (comme des formes d'animaux pour un complément destiné aux enfants).

Un autre exemple de réalisation sous forme de pastilles à gomme fondante a été réalisé avec les ingrédients en quantités suivantes (% en poids) :
- Eau : 14,75 % ;
- Sirop de glucose : 13,82 % ;
- Sucre (sucre cristallisé et sucre glace) : 65,42 % ;
- Gélatine : 1,44 % ;
- Arôme : 0,35 % ;
- Lactase fongique : 4,22 %.

Il s'agit ci-dessus des quantités avant la ou les opérations d'évaporation de l'eau. Après la ou les opérations d'évaporation de l'eau, la composition du produit final présente les quantités suivantes (% en poids) :
- Eau : 5,15 % ;
- Sirop de glucose : 15,37% ;
- Sucre (sucre cristallisé et sucre glace) : 72,79 % ;
- Gélatine : 1,61 % ;
- Arôme : 0,38 % ;
- Lactase fongique : 4,7 %.

D'autres exemples de réalisation répondant aux caractéristiques de l'invention ont été mis en oeuvre. Plus particulièrement, des mélanges ont a été réalisés avec les ingrédients en quantités suivantes (% en poids) :
- Eau : 14,0 % à 16,5 % ;
- Sirop de glucose : 13,5% à 15,5 % ;
- Sucre (sucre cristallisé et sucre glace) : 62,5 % à 65,5 % ;
- Gélatine : 1 % à 1,45 % ;
- Arôme : 0,08 % à 0,4 % ;
- Lactase fongique : 2,1 % à 5 %.

Il s'agit ci-dessus des quantités avant la ou les opérations d'évaporation de l'eau. Après la ou les opérations d'évaporation de l'eau, les compositions de produits finaux présentent les quantités suivantes (% en poids) :
- Eau : 3,5 à 8 % ;
- Sirop de glucose : 15,3 % à 20 % ;
- Sucre (sucre cristallisé et sucre glace) : 69 % à 75 % ;
- Gélatine : 1,5 % à 1,7 % ;
- Arôme : 0,1 % à 0,45 % ;
- Lactase fongique : 2,25 % à 6 %.

Optionnellement, chaque mélange peut comprendre un colorant pour rendre l'aspect du produit final plus attractif. Le mélange avant évaporation de l'eau peut notamment contenir de 0,1 % à 1,5 % de colorant. Le produit final après évaporation peut contenir de 0,1 % à 2 % de colorant. Il convient alors d'enlever une quantité d'eau correspondante.

La présente invention permet de conserver une activité lactase pendant une longue durée dans un complément alimentaire. En effet, la lactase présente dans la composition de l'invention ne se dégrade pas ou quasiment pas durant plusieurs mois à température ambiante (pendant une période allant au-delà de six mois). Les problèmes de production industrielle et de commercialisation concernant la DLUO (date limite d'utilisation optimale) et/ou du surdosage excessifs sont résolus.

De plus, l'aspect sous forme de gomme fondante de la composition de l'invention est très agréable à la consommation. Les arômes de la composition donnent à la gomme fondante un semblant de friandise consommable. Le complément alimentaire de l'invention convient donc à la consommation avant, après ou entre les repas tout au long d'une journée. Ceci permet aux personnes ayant une intolérance au lactose d'ingérer régulièrement une dose efficace de lactase. Les produits laitiers et tout autre produit contenant du lactose peuvent donc être consommés normalement. Le complément alimentaire de l'invention est donc un moyen efficace pour lutter contre l'intolérance au lactose.

## Revendications

1. Composition de complément alimentaire confectionnée sous forme de gomme fondante, comprenant en poids:
- 3% à 8% d'eau ;
- 14% à 20% de sirop de glucose ;
- 60% à 80% de sucre ;
- 1% à 3% de gélatine ;
- 0,1% à 2% d'une ou plusieurs substances arômatisantes ;
- 2% à 6% de lactase fongique issue de *Aspergillus oryzae* présentant une activité enzymatique de 100000 unités par gramme d'enzyme
dans laquelle le sucre comprend en poids 0,15% en sucre glace et le reste en sucre cristallisé.

2. Composition selon la revendication 1, comprenant en outre de 0,1% à 2% de colorant.

3. Procédé de production de complément alimentaire selon l'une des revendications 1 et 2, comprenant les étapes suivantes :
a. Mélanger de l'eau, du sirop de glucose, du sucre et de la gélatine pour obtenir une première formulation ;
b. Ajouter de la lactase fongique à la première formulation pour obtenir une deuxième formulation, la lactase fongique étant issue de *Aspergillus oryzae* et présentant une activité enzymatique de 100000 unités par gramme d'enzyme ;
c. Réguler en cuve tampon la deuxième formulation par ajout de sucre pour obtenir une troisième formulation ;
d. Ajouter une ou plusieurs substances arômatisantes ;
e. Aérer la troisième formulation dans un foisonneur de manière à former une gomme fondante ;
f. Sécher ladite gomme fondante par évaporation d'une partie de l'eau ;
dans lequel les quantités en poids de l'eau, du sirop de glucose, du sucre, de la gélatine, de la lactase fongique et de la ou les substances arômatisantes dans les étapes a. à d. sont choisies de manière à obtenir une composition pour complément alimentaire après le séchage de l'étape f. comportant 3% à 8% d'eau, 14% à 20% de sirop de glucose, 60% à 80% de sucre, 1% à 3% de gélatine, 0,1% à 2% d'une ou plusieurs substances arômatisantes, et 2% à 6% de lactase fongique.

4. Procédé selon la revendication 3, dans lequel l'étape a. comprend les sous-étapes de :
a1. Mélanger une partie de l'eau et une partie du sirop de glucose à une température inférieure ou égale à 80°C ;
a2. Ajouter au mélange de l'étape a1. du sucre et mélanger à une température d'environ 80°C ;
a3. Ajouter le restant du sirop de glucose au mélange de l'étape a2. et mélanger à une température comprise entre 80°C et 100°C ;
a4. Mélanger la gélatine avec le restant de l'eau à une température d'environ 95°C ;
a5. Ajouter le mélange obtenu à l'étape a4. au mélange obtenu à l'étape a3. à une température d'environ 90°C.

5. Procédé selon l'une des revendications 3 et 4, dans lequel l'ajout de la lactase fongique à l'étape b. est réalisé à une température d'environ 45°C.

6. Procédé selon l'une des revendications 3 à 5, dans lequel la régulation à l'étape c. comprend l'ajout de sucre glace.

7. Procédé selon l'une des revendications 3 à 6, dans lequel l'étape d. est réalisée sensiblement avec l'étape b.

8. Procédé selon l'une des revendications 3 à 6, dans lequel l'étape d. est réalisée après l'étape e.

9. Procédé selon l'une des revendications 3 à 8, dans lequel l'étape f. de séchage comprend en outre l'étape de :
f1. Confectionner ladite troisième formulation par une ou plusieurs opérations choisies parmi le moulage/démoulage dans des moules d'amidon, le coulage, le séchage et le dépoudrage.

10. Procédé selon l'une des revendications 3 à 9, comprenant en outre l'étape de :
g. Stocker ladite troisième formulation.

## Patentansprüche

1. Nahrungsergänzungsmittelzusammensetzung, hergestellt in Form von Schmelzgummi, umfassend nach Gewicht:
- zu 3 % bis 8 % Wasser;
- zu 14 % bis 20 % Glukosesirup;
- zu 60 % bis 80 % Zucker;
- zu 1 % bis 3 % Gelatine;
- zu 0,1 % bis 2 % ein oder mehrere Aromastoffe;
- zu 2 % bis 6 % Pilzlaktase aus *Aspergillus oryzae* mit einer enzymatischen Aktivität von 100.000 Einheiten pro Gramm Enzym,
wobei der Zucker zu 0,15 Gew.-% aus Puderzucker und im Übrigen aus kristallisiertem Zucker besteht.

2. Zusammensetzung nach Anspruch 1, weiterhin umfassend 0,1 % bis 2 % Farbstoff.

3. Verfahren zur Herstellung eines Nahrungsergänzungsmittels nach einem der Ansprüche 1 und 2, umfassend folgende Schritte:
a. Mischen von Wasser, Glukosesirup, Zucker und Gelatine, um eine erste Formulierung zu erhalten;
b. Zugabe von Pilzlaktase zur ersten Formulierung, um eine zweite Formulierung zu erhalten, wobei die Pilzlaktase aus *Aspergillus oryzae* stammt und eine enzymatische Aktivität von 100.000 Einheiten pro Gramm Enzym aufweist;
c. Regulieren der zweiten Formulierung in einem Puffertank durch Zugabe von Zucker, um eine dritte Formulierung zu erhalten;
d. Zugabe eines oder mehrerer Aromastoffe;
e. Belüften der dritten Formulierung in einer Quellvorrichtung, um einen Schmelzgummi zu bilden;
f. Trocknen des Schmelzgummis durch Verdampfen eines Teils des Wassers;
wobei die Gewichtsmengen an Wasser, Glukosesirup, Zucker, Gelatine, Pilzlaktase und dem oder den Aromastoffen in den Schritten a bis d so gewählt werden, dass nach dem Trocknen in Schritt feine Nahrungsergänzungszusammensetzung entsteht, die zu 3 % bis 8 % Wasser, zu 14 % bis 20 % Glukosesirup, zu 60 % bis 80 % Zucker, zu 1 % bis 3 % Gelatine, zu 0,1 % bis 2 % einen oder mehrere Aromastoffe und zu 2 % bis 6 % Pilzlaktase umfasst.

4. Verfahren nach Anspruch 3, wobei Schritt a folgende Unterschritte umfasst:
a1. Mischen eines Teils des Wassers und eines Teils des Glukosesirups bei einer Temperatur von weniger oder gleich 80 °C;
a2. Zugabe von Zucker zu der Mischung aus Schritt a1, und Mischen bei einer Temperatur von etwa 80 °C;
a3. Zugabe des restlichen Glukosesirups zu der Mischung aus Schritt a2 und
Mischen bei einer Temperatur zwischen 80 °C und 100 °C;
a4. Mischen der Gelatine mit dem restlichen Wasser bei einer Temperatur von etwa 95 °C;
a5. Zugabe der in Schritt a4 erhaltenen Mischung zu der in Schritt a3 erhaltenen Mischung bei einer Temperatur von etwa 90 °C.

5. Verfahren nach einem der Ansprüche 3 und 4, wobei die Zugabe der Pilzlaktase in Schritt b bei einer Temperatur von ca. 45 °C erfolgt.

6. Verfahren nach einem der Ansprüche 3 bis 5, wobei die Regulierung in Schritt c die Zugabe von Puderzucker umfasst.

7. Verfahren nach einem der Ansprüche 3 bis 6, wobei Schritt d im Wesentlichen zusammen mit Schritt b ausgeführt wird.

8. Verfahren nach einem der Ansprüche 3 bis 6, wobei Schritt d nach Schritt e ausgeführt wird.

9. Verfahren nach einem der Ansprüche 3 bis 8, wobei Schritt f zum Trocknen weiterhin folgenden Schritt umfasst:
f1. Herstellen der dritten Formulierung durch einen oder mehrere Vorgänge, ausgewählt aus Formen/Entformen in Stärkeformen, Gießen, Trocknen und Entfernen von Pulver.

10. Verfahren nach einem der Ansprüche 3 bis 9, umfassend weiterhin folgenden Schritt:
g. Lagern der dritten Formulierung.

## Claims

1. Composition of dietary supplement made in the form of melting gum, comprising by weight:
- 3 % to 8 % of water;
- 14 % to 20 % of glucose syrup;
- 60 % to 80 % of sugar;
- 1 % to 3 % of gelatin;
- 0.1 % to 2 % of one or more flavoring substances;
- 2 % to 6% of fungal lactase from *Aspergillus oryzae* having an enzymatic activity of 100000 units per gram of enzyme.
wherein the sugar comprises 0.15% by weight of powdered sugar and the rest of crystallized sugar.

2. Composition according to claim 1, further comprising 0.1% to 2% of colorant.

3. Process for the production of a dietary supplement comprising the following steps:
a. Mixing water, glucose syrup, sugar and gelatin for obtaining a first formulation;
b. Adding fungal lactase to the first formulation for obtaining a second formulation, wherein the fungal lactase comes from *Aspergillus oryzae* and has an enzymatic activity of 100000 units per gram of enzyme;
c. Adjusting the second formulation in a buffer vat by adding sugar in order to obtain a third formulation;
d. Adding one or more flavoring substances;
e. Aerating the third formulation in a multiplier in order to form a melting gum;
f. Drying said melting gum by evaporation part of the water;
wherein the quantities by weight of the water, glucose syrup, sugar, gelatin, fungal lactase and of the flavoring substance(s) in steps a. to d. are selected so that to obtain a composition of a dietary supplement after the drying of step f. comprising 3% to 8% of water; 14% to 20% of glucose syrup; 60% to 80% of sugar; 1% to 3% of gelatin; 0.1% to 2% of one or more flavoring substances, and 2% to 6% of fungal lactase.

4. The process according to Claim 3, wherein the step a. comprises the sub-steps of:
a1. Mixing part of the water and part of the glucose syrup at a temperature lower or equal to 80°C ;
a2. Adding sugar to the mixture of step a1. and mixing at a temperature of approximately 80°C;
a3. Adding the remainder of the glucose syrup to the mixture of step a2. and
mixing at a temperature between 80°C and 100°C;
a4. Mixing the gelatin with the remainder of the water at a temperature of approximately 95°C;
a5. Adjusting the mixture obtained in step a4. to the mixture obtained in step a3. at a temperature of approximately 90°C.

5. The process according to one of Claims 3 and 3, wherein the addition of fungal lactase in step b. is carried out at a temperature of approximately 45°C.

6. The process according to one of Claims 3 to 5, wherein the adjustment at step c. comprises the addition of powdered sugar.

7. The process according to one of Claims 3 to 6, wherein step d. is carried out before step b.

8. The process according to one of Claims 3 to 6, wherein step d. is carried out before step e.

9. The process according to one of Claims 3 to 8, wherein the drying step f. also comprises the step of:
f1. Making said third formulation by one or several operations selected from molding/demolding in molds of starch, casting, drying and depowdering.

10. The process according to one of Claims 3 to 9, also comprising the step of:
g. Storing said third formulation.
